# EUROPÄISCHE PATENTSCHRIFT

(11) **EP 4 017 425 B1**
(45) Veröffentlichungstag und Bekanntmachung des Hinweises auf die Patenterteilung: **25.02.2026**
(21) Anmeldenummer: 20754740.7
(22) Anmeldetag: 11.08.2020
(51) Int. Cl.: A61F 2/50, A61F 2/76, A61F 2/60, A61F 2/48, A61B 5/055, A61B 5/00

(54) **VERFAHREN ZUM HERSTELLEN EINES PROTHESENSCHAFTES**
METHOD FOR MANUFACTURING A PROSTHESIS SOCKET
PROCÉDÉ DE FABRICATION D'UNE EMBOÎTURE DE PROTHÈSE

(30) Priorität: 20.08.2019 DE 102019122374
(43) Veröffentlichungstag der Anmeldung: 29.06.2022
(73) Patentinhaber: Ottobock SE & Co. KGaA, 37115 Duderstadt (DE)
(72) Erfinder: GLORIEUX, Dries, 3920 Lommel (BE); BUSSIEK-CILLIEN, Kai-Hendrik, 37075 Göttingen (DE); SCHÖNEMEIER, Mark, 37077 Göttingen (DE)
(74) Vertreter: Gramm, Lins & Partner Patent- und Rechtsanwälte PartGmbB
(86) Internationale Anmeldenummer: PCT/EP2020/072509
(87) Internationale Veröffentlichungsnummer: WO 2021/032538

(56) Entgegenhaltungen:
- WO-A1-2019/157486
- WO-A2-2007/144608
- US-A1- 2017 290 685

## Beschreibung

Die Erfindung betrifft ein Verfahren zum Herstellen eines orthopädietechnischen Produktes für ein Körperteil eines Patienten und ein Verfahren zum Erzeugen von Produktionsdaten für ein orthopädietechnisches Produkt.

Orthopädietechnische Produkte sind im Sinne der vorliegenden Erfindung insbesondere Orthesen und Prothesen. Orthesen sind Produkte, die ein Körperteil des Patienten, beispielsweise ein Gelenk, stützen, unterstützen, schützen oder in der Bewegungsfreiheit einschränken, um eine Überbeanspruchung zu vermeiden. Prothesen hingegen ersetzen nicht oder nicht mehr vorhandene Körperteile des Patienten.

Unter einem Patienten wird im Folgenden jeder Benutzer des orthopädietechnischen Produktes verstanden. Es handelt sich folglich um den Träger des herzustellenden Produktes.

Jedes orthopädietechnische Produkt wird an einem Körperteil des Patienten angeordnet. Dabei muss es nicht zwangsläufig mit der Haut des Patienten in Kontakt kommen. Orthesen werden beispielsweise häufig über der Kleidung getragen, sodass diese, beispielsweise eine Hose, sich zwischen dem orthopädietechnischen Produkt und der Haut des Patienten befindet. Dennoch wird beispielsweise eine Knie-Orthese am Knie oder am Bein des Patienten befestigt. Eine Prothese verfügt immer über ein mit einem Amputationsstumpf oder einem anderen Körperteil verbundenes Schnittstellenelement, das an dem jeweiligen Körperteil befestigt wird. Bei einer Beinprothese wird beispielsweise ein Prothesenschaft verwendet, der die Schnittstelle zwischen der Prothese und dem Amputationsstumpf darstellt. In diesem Fall wäre der Amputationsstumpf der Körperteil des Patienten.

Ein Prothesenschaft für einen Amputationsstumpf wird in der Regel aus einem rigiden und starren Material, beispielsweise einem faserverstärkten Kunststoff, hergestellt und bildet einen wichtigen Teil des Interfaces zwischen dem Amputationsstumpf und der Prothese, die am Prothesenschaft angeordnet wird. Insbesondere für Beinprothesen, die an einem Amputationsstumpf, beispielsweise einem Oberschenkelstumpf, angeordnet werden sollen, werden entsprechende Prothesenschäfte seit langem verwendet. Die Erfindung ist jedoch nicht auf diese Art Prothesenschäfte beschränkt. Prothesenschäfte der hier beschriebenen Art können auch für Armprothesen oder Unterschenkelprothesen verwendet werden.

Insbesondere Prothesenschäfte für Beinamputierte sind im täglichen Gebrauch besonderen Belastungen ausgesetzt. Beim Gehen lastet das gesamte Gewicht des Patienten auf dem Prothesenschaft und damit insbesondere auf dem Amputationsstumpf, der in dem Prothesenschaft angeordnet wird. Es ist daher von größter Wichtigkeit, den Prothesenschaft möglichst optimal an die individuellen Gegebenheiten und Bedürfnisse des Patienten anzupassen.

Aus dem Stand der Technik sind daher eine Reihe unterschiedlicher Verfahren bekannt, mit denen die Form und Ausgestaltung des Prothesenschaftes dem Amputationsstumpf unter Last angepasst werden sollen. Dazu wird in der Regel der Amputationsstumpf abgeformt, um einen Rohling und Grundkörper zu erhalten, auf dem der Prothesenschaft aufgebaut wird. Dabei dominiert noch immer das Abformen des Stumpfes mittels Gips zur Erstellung einer Negativform des Stumpfes. Neben den geringen Kosten hat diese Methode den Vorteil, dass geübte Orthopädietechniker über eine gezielte Formung des Amputationsstumpfes beim Gipsen bereits Einfluss auf die Form des späteren Prothesenschaftes nehmen können. Dies ist insbesondere dann von Vorteil, wenn der spätere Schaft spezifische Belastungszonen aufweisen soll.

Aus der US 2017/290685 A1 ist beispielsweise ein Verfahren bekannt, bei dem der Amputationsstumpf mittels eines 3D-Scanners erfasst und die so gewonnenen Daten mit einer speziellen Anzeigevorrichtung dargestellt werden. Dadurch kann das so dargestellte 3-dimensionale Objekt frei gedreht und betrachtet werden. Eine ähnliche Art der Darstellung ist aus der WO 2019/157486 A1 bekannt.

Um eine möglichst gleichmäßige Belastung auf den Amputationsstumpf aufbringen zu können, sind vermehrt Methoden entwickelt worden, die einen gleichmäßigen Druck auf den Amputationsstumpf ausüben, während die Form des Amputationsstumpfes abgenommen wird. Dabei wird der Stumpf beispielsweise in einem wassergefüllten Volumen angeordnet, sodass das Wasser einen gleichmäßigen Druck ausüben kann. Alternativ zu einem Wasserbad kann auch ein mit Sand gefülltes Volumen verwendet werden. Nachteilig ist jedoch, dass derartige Methoden bauartbedingt wenig transportabel sind und somit im klinischen Alltag kaum angewendet werden.

Optische Scanverfahren, bei denen der Amputationsstumpf durch optische Kameraeinrichtungen vermessen wird, sind aus dem Stand der Technik bekannt Doch selbst wenn ein solches Verfahren verwendbar ist, wird der Amputationsstumpf lediglich statisch vermessen. Eine Erfassung von Daten während der Bewegung des Amputationsstumpfes ist in der Regel nicht möglich. Zudem muss die Scan-Vorrichtung zumeist manuell um das zu erfassende Körperteil herumgeführt werden, was aufwendig und wenig komfortabel ist. Außerdem ist es in der Regel nicht möglich, den Amputationsstumpf beispielsweise mit den Händen zu verformen, indem ein Druck aufgebaut wird, und den so verformten Amputationsstumpf zu erfassen.

Bei allen genannten Verfahren, die aus dem Stand der Technik bekannt sind, besteht der Nachteil, dass allenfalls die äußere Form des Amputationsstumpfes in die Formgebung des Prothesenschaftes einfließt. Die Positionierung von besonders gepolsterten oder zu entlastenden Bereichen ist nur über die Erfahrung, die der Orthopädietechniker gesammelt hat, möglich. Besonders individuelle Anforderungen oder Gegebenheiten, die durch Besonderheiten des Amputationsstumpfes gegeben sind, können nur schwer einbezogen werden. Bei Scan-Verfahren besteht zudem der Nachteil, dass der Benutzer des Verfahrens, beispielsweise ein Orthopädietechniker das Ergebnis des Scans nicht direkt am Stumpf ändern und anpassen kann. Er verliert daher die herkömmlicherweise vorhandene Möglichkeit, den Stumpf abzuformen und anzupassen. Eine nachträgliche Veränderung und Anpassung des Scans ist zwar mittels einer elektronischen Datenverarbeitungseinrichtung, beispielsweise einem Computer, Tablet oder Laptop möglich, bietet dem Benutzer jedoch nicht das haptische Feedback, das beim Andrücken und Abformen am Stumpf erfolgt.

Ein weiterer Nachteil besteht darin, dass die Formgebung des Amputationsstumpfes, wie bereits dargelegt, lediglich anhand von statischen Daten, also beispielsweise der äußeren Form des unbelasteten Stumpfes, einfließt. Daher wird zunächst ein Testschaft erzeugt, der am Patienten getestet und von diesem getragen werden muss. Auf diese Weise wird geprüft, ob der Schaft den Anforderungen gerecht wird oder ob Änderungen nötig sind. Gegebenenfalls muss die Form des Testschaftes an eine dynamische Situation angepasst werden, wenn der Testschaft Probleme und nicht optimale Formgebungen offenbart. Dies ist zeitaufwendig und kostenintensiv.

Insbesondere bei Prothesenschäfte, jedoch auch bei anderen orthopädietechnischen Produkten, beispielsweise Orthesen, werden Bauteile oder die gesamten Produkte optimaler Weise individuell an die körperlichen Gegebenheiten des Patienten angepasst. In vielen Fällen ist dafür notwendig, den Körperteil exakt zu vermessen, was mit den oben ausgeführten Problemen verbunden ist.

Der Erfindung liegt daher die Aufgabe zugrunde, ein Verfahren zum Herstellen eines orthopädietechnischen Produktes für ein Körperteil eines Patienten vorzuschlagen, das diese Nachteile behebt oder zumindest mildert.

Die Erfindung löst die gestellte Aufgabe durch ein Verfahren zum Herstellen eines orthopädietechnischen Produktes für ein Körperteil eines Patienten, wobei das Verfahren folgende Schritte aufweist:
a) Bereitstellen von Körperteildaten, die Informationen über einen inneren Aufbau des Körperteils enthalten,
b) Erfassen einer aktuellen Blickrichtung, aus der ein Benutzer des Verfahrens das Körperteil sieht,
c) Darstellen der Körperteildaten aus der aktuellen Blickrichtung mittels der Anzeigeeinrichtung, so dass der Benutzer das Körperteil und die Körperteildaten überlagert sieht,
d) Verformen und/oder Abformen des Körperteils,
e) Erfassen von Bilddaten des verformten und/oder abgeformten Körperteils mittels einer Kamera und/oder einer Scanvorrichtung zumindest auch aus der aktuellen Blickrichtung, die mittels der Anzeigeeinrichtung den Körperteildaten überlagert dargestellt werden,
f) Erzeugen von Produktionsdaten für das orthopädietechnische Produkt auf der Grundlage der dargestellten Daten und

Bereitstellen eines auf der Grundlage der Produktionsdaten hergestellten orthopädietechnischen Produktes.

Durch das erfindungsgemäße Verfahren wird es möglich, Körperteildaten, die Informationen über den inneren Aufbau des Körperteils beinhalten, in die Produktion des orthopädietechnischen Produktes einzubeziehen.

Ein Benutzer des Verfahrens, also jemand, beispielsweise ein Orthopädietechniker, der das erfindungsgemäße Verfahren durchführt, kann auf der Anzeigeeinrichtung, die beispielsweise ein Monitor sein kann, Informationen über den inneren Aufbau des Körperteils in Form der Körperteildaten erkennen und gleichzeitig die äußere Kontur und Form des Körperteils sehen. So kann er die Form des orthopädietechnischen Produktes optimal an die gezeigten Informationen anpassen. Dies geschieht beim Erzeugen der Produktionsdaten, was vorzugsweise virtuell geschieht. Mithilfe einer elektronischen Datenverarbeitungseinrichtung, die vorzugsweise auch die Darstellung der mittels der Anzeigeeinrichtung angezeigten Daten steuert, lässt sich elektronisch ein orthopädietechnisches Produkt erstellen, das den angezeigten Daten und der dadurch dargestellten Situation des Patienten Rechnung trägt.

In bevorzugten Ausgestaltungen, die weiter unten näher beschrieben werden, beinhaltet der Schritt des Erzeugens von Produktionsdaten weitere Teilschritte, sodass die Produktionsdaten nicht einfach auf der Grundlage der Körperteildaten erstellt werden. Bevorzugt kann der Benutzer des Verfahrens, vorzugsweise der Orthopädietechniker, das Körperteil, beispielsweise einen Amputationsstumpf, verformen oder abformen, sodass geänderte Körperteildaten als die ursprünglich bereitgestellten Körperteildaten den Produktionsdaten zugrunde gelegt werden. Bevorzugt gibt der Benutzer des Verfahrens die angezeigten Daten zur Erstellung von Produktionsdaten frei.

Die so erstellten und erzeugten Produktionsdaten werden dann einer Produktionsstelle zur Verfügung gestellt, die orthopädietechnische Produkteherstellt. Dies kann beispielsweise dadurch geschehen, dass auf der Grundlage der Produktionsdaten eine Negativform oder eine Positivform eines Amputationsstumpfes erstellt wird, die als Grundlage für einen herzustellenden Prothesenschaft verwendet wird. So kann beispielsweise auf eine Positivform des Amputationsstumpfes ein kohlefaserverstärkter Prothesenschaft aufgebaut werden, indem beispielsweise mit einem Kunstharz getränkte Kohlefasermatten aufgelegt werden, die anschließend in einem an sich bekannten Verfahren ausgehärtet werden. Alternativ dazu können die Produktionsdaten auch einem 3-D-Drucker zur Verfügung gestellt werden, der dann aus einem geeigneten Kunststoffmaterial den Prothesenschaft 3-dimensional druckt. Dabei sind prinzipiell alle additiven Fertigungsmethoden geeignet, durch die für einen Prothesenschaft geeignete Werkstoffe verarbeitet werden können.

Alternativ dazu können beispielsweise auch Schienensysteme, Gelenke, Schalen oder sonstige Bauteile einer Orthese hergestellt werden, die den individuellen Gegebenheiten des Patienten, beispielsweise Größen und Längen bestimmter Körperteile, wie beispielsweise Arme und Beine, Position und Orientierung unterschiedlicher Körperteile zueinander und Bewegungsmöglichkeiten und Bewegungseinschränkungen beispielsweise von Gelenken Rechnung tragen. Dies ist insbesondere bei Schalen von Vorteil, die direkt an einen Körperteil des Patienten angefordert und angepasst werden und beim späteren Gebrauch der orthopädietechnischen Produkte mit diesem Körperteil direkt in Kontakt kommen.

Wichtig ist, dass der Benutzer des Verfahrens die Körperteildaten, die Informationen über den inneren Aufbau des Körperteils enthalten, ebenso aus der aktuellen Blickrichtung oder zumindest nahezu aus der aktuellen Blickrichtung sieht wie das Körperteil. So lassen sich beispielsweise die Enden der Knochen, Muskelverläufe und Nervenbahnen darstellen und bei der Herstellung des orthopädietechnischen Produktes und der Erzeugung der Produktionsdaten für dieses Produkt berücksichtigen.

Das Körperteil kann ein Arm, beispielsweise ein Oberarm oder ein Unterarm, ein Bein, beispielsweise ein Oberschenkel oder ein Unterschenkel, oder ein Gelenk, beispielsweise ein Hüftgelenk, ein Knie, ein Knöchelgelenk oder ein Ellenbogen sein. Insbesondere für Prothesen und Prothesenschäfte kann das Körperteil auch ein Amputationsstumpf, beispielsweise ein Oberschenkelstumpf, ein Unterschenkelstumpf oder ein Armstumpf sein.

Vorzugsweise sind die Körperteildaten 3-dimensionale Körperteildaten.

In einer bevorzugten Ausgestaltung des Verfahrens werden mittels der Anzeigeeinrichtung die Körperteildaten dargestellt, während der Benutzer des Verfahrens direkt auf das Körperteil blickt. Dies ist beispielsweise mittels einer entsprechenden Brille (Virtual Reality (VR)-Brille, Augmented Reality (AR)-Brille) oder einem anderen technischen Gerät möglich. Die Anzeigeeinrichtung ist in dem besonders bevorzugten Fall eine AR-Brille teiltransparent ausgebildet. Dies bedeutet, dass optische Daten, insbesondere die Körperteildaten angezeigt werden können und der Benutzer gleichzeitig durch die Anzeigeeinrichtung hindurch das Körperteil sehen kann. An der Anzeigeeinrichtung ist vorzugsweise eine Erfassungseinrichtung angeordnet, durch die die aktuelle Blickrichtung, in die der Benutzer der Anzeigeeinrichtung blickt, erfassbar ist.

Alternativ oder zusätzlich dazu können die Körperteildaten auch auf das Körperteil selbst projiziert werden. Die Anzeigeeinrichtung ist dann ein Projektor, der die Körperteildaten vorzugsweise entlang der aktuellen Blickrichtung auf den Körperteil, beispielsweise einen Amputationsstumpf, projiziert.

Diese Ausgestaltungen haben den Vorteil, dass der Benutzer des Verfahrens, der bisher Verfahren aus dem Stand der Technik verwendet hat, bei denen er mit seinen Händen direkt das Körperteil angedrückt und abgeformt hat, sich nicht umgewöhnen muss. Er kann weiterhin direkt am Körperteil arbeiten und dabei auf seine Hände und das Körperteil blicken. Es ist nicht notwendig, eine separate Anzeigeeinrichtung zu verwenden, die beispielsweise ein Monitor oder Bildschirm sein kann.

Alternativ oder zusätzlich dazu werden Bilddaten des Körperteils erfasst, wozu beispielsweise eine Kameraeinrichtung verwendet wird. Die aktuelle Blickrichtung entspricht dann der Blickrichtung der Kamera auf das Körperteil. Der Benutzer des Verfahrens blickt auf die Anzeigeeinrichtung, beispielsweise in Form eines Monitors oder Bildschirms und sieht das Körperteil aus der Blickrichtung der Kameraeinrichtung. Um ein möglichst realistisches Bild zu erhalten ist es notwendig, auf der Anzeigeeinrichtung auch die Körperteildaten aus der aktuellen Blickrichtung anzuzeigen. Da es sich dabei um Körperteildaten handelt, ist die elektronische Datenverarbeitungseinrichtung in der Lage, zu errechnen, welche Daten in welcher Relation zueinander angezeigt werden können.

Alternativ oder zusätzlich können Bilddaten auch mittels einer Scanvorrichtung erfasst werden. Dies kann beispielsweise ein Scanliner oder ein Scanhandschuh sein. Handelt es sich bei dem Körperteil beispielsweise um einen Amputationsstumpf, kann ein Scanliner verwendet werden, der über den Amputationsstumpf gezogen wird und in der Lage ist, seine eigene geometrische Form zu erfassen. Dies kann beispielsweise über Dehnungsmessstreifen innerhalb des Liners geschehen, die den Abstand zwischen genau festgelegten Punkten des Liners bestimmen. Geschieht dies über einen ausreichend großen Anteil des Liners, vorzugsweise über den gesamten Liner, lässt sich die Kontur und geometrische Form des Liners und damit auch die geometrische Form und Kontur des im Liner enthaltenen Amputationsstumpfes bestimmen. Alternativ oder zusätzlich dazu können auch bei vorhandenen Körperteilen, beispielsweise Armen, Beinen, Füßen oder Gelenken Scanhandschuhe zum Einsatz kommen. Diese verfügen über Sensoren, die beispielsweise Absolut-Positionen messen und bestimmen können, sodass die geometrische Form und die Außenkontur des Körperteils erfasst und vermessen werden können, indem beispielsweise die Haut des Körperteils vom Benutzer des Verfahrens überstrichen wird, während er einen Scanhandschuh trägt.

Entsprechende Sensoren, die im Scanhandschuh oder im Scanliner verwendet werden, sind beispielsweise Fibre-Bragg-Sensoren, Ultraschallsensoren oder magnetische Sensoren, durch die die äußere Form des Körperteils erfassbar ist. Diese Form kann zudem aktualisiert werden, wenn der Benutzer des Verfahrens, beispielsweise ein Orthopädietechniker, der Körperteil verformt. So könnte beispielsweise über Drucksensoren ein Anpressdruck ermittelt werden.

Mittels einer Scanvorrichtung erfasste Bilddaten werden im Folgenden auch Konturdaten genannt.

Handelt es sich bei der Kameraeinrichtung, durch die die Bilddaten des Körperteils erfasst werden, um eine einzelne Kamera, entspricht die Blickrichtung der Richtung, aus der die Kamera das Körperteilerfasst. Dies beinhaltet neben der Richtung, aus der die Kamera elektromagnetische Strahlung aufnehmen kann, auch die Orientierung des Körperteils relativ zur Kamera. Die Position und Orientierung des Körperteils relativ zur Kamera sind bekannt, sodass beide in einem einzigen Koordinatensystem positioniert werden können. Damit ist auch die Blickrichtung, aus der die Bilddaten dargestellt werden, vorgegeben. Handelt es sich bei der Kameraeinrichtung nicht um eine einzelne Kamera, sondern um eine Einrichtung mit mehreren Kameras, sodass beispielsweise eine 3-dimensionale Erfassung des Körperteils möglich ist, kann die Blickrichtung frei oder zumindest nahezu frei gewählt werden. Dazu können Bedienelemente vorhanden sein, durch die der Benutzer des Verfahrens die Blickrichtung je nach Bedarf ändern kann.

Bevorzugt wird eine teiltransparente Anzeigeeinrichtung verwendet, durch die neben den Körperteildaten zusätzlich auch Bilddaten und/oder Konturdaten des Körperteils angezeigt werden. Diese Bilddaten werden folglich vom Benutzer des Verfahrens erfasst. Da die Anzeigeeinrichtung teiltransparent ist, kann er zusätzlich direkt auf das Körperteil blicken. Es ist daher von Vorteil, wenn die Bilddaten, die angezeigt werden, zusätzliche Informationen enthalten. Es kann vorzugsweise erreicht werden, indem die Bilddaten beispielsweise in einem Frequenzbereich erfasst wurden, der durch das menschliche Auge nicht wahrnehmbar ist. So kann beispielsweise eine Infrarot-Kamera verwendet werden, um ein Wärmebild des Körperteils zu generieren, wodurch beispielsweise Rückschlüsse auf die Durchblutung unterschiedlicher Bereiche des Körperteils gezogen werden können. Alternativ oder zusätzlich dazu können Daten über die Durchblutung auch Teil der Körperteildaten sein.

Vorzugsweise werden zum Erzeugen der Produktionsdaten zunächst Produktdaten auf der Grundlage der dreidimensionalen Körperteildaten und/oder der erfassten Bilddaten und/oder Konturdaten erzeugt, auf deren Grundlage die Produktionsdaten erzeugt werden. Die 3-dimensionalen Produktdaten werden bevorzugt durch die elektronische Datenverarbeitungseinrichtung erzeugt. Selbst wenn eine teiltransparente Anzeigeeinrichtung verwendet wird, durch die lediglich die Körperteildaten angezeigt werden, ist es in diesem Fall von Vorteil, Bilddaten und/oder Konturdaten des Körperteiles über eine Kameraeinrichtung zu erfassen, die der Datenverarbeitungseinrichtung übermittelt werden. Auf diese Weise kann die Datenverarbeitungseinrichtung die 3-dimensionalen Produktdaten auf der Grundlage der angezeigten dreidimensionalen Körperteildaten und nicht angezeigter Bilddaten und/oder Konturdaten des Körperteils generieren. Dabei ist jedoch wichtig, dass die Bilddaten aus der aktuellen Blickrichtung aufgenommen werden.

Der Benutzer des Verfahrens kann Änderungen an den Produktdaten vornehmen. Dies kann beispielsweise geschehen, indem das Körperteil, beispielsweise ein Amputationsstumpf, abgeformt oder verformt wird. Alternativ oder zusätzlich dazu kann der Benutzer, beispielsweise ein Orthopädietechniker, Markierungen auf den Körperteil aufbringen oder Bereiche beispielsweise durch Handgesten markieren, die von einer Kamera erfasst und von einer elektronischen Datenverarbeitungseinrichtung erkannt und verarbeitet werden können. So ist es beispielsweise möglich, Bereiche zu markieren, in denen später eine Polsterung angeordnet werden soll. Auch unterschiedliche Materialien, die bei dem herzustellenden Produkt verwendet werden sollen, können auf diese Weise unterschiedlichen Bereichen zugeordnet werden.

Vorzugsweise wurden die Körperteildaten, insbesondere die 3-dimensionalen Körperteildaten durch ein bildgebendes medizinisches Verfahren gewonnen und sind beispielsweise in einem elektronischen Datenspeicher, auf den eine elektronische Datenverarbeitungseinrichtung zugreifen kann, abgelegt. Bei dem medizinischen Verfahren kann es sich beispielsweise um ein CT-Verfahren (CT: Computertomografie), ein MRT-Verfahren (MRT: Magnetresonanz Tomografie) oder ein sonstiges bildgebendes Verfahren handeln. Diese Daten sind oftmals ohnehin vorhanden, da sie nach der Operation aufgenommen und erfasst wurden. Alternativ oder zusätzlich dazu können entsprechenden Körperteildaten im Rahmen des hier beschriebenen Verfahrens aufgenommen und anschließend bereitgestellt werden.

Alternativ oder zusätzlich dazu können als Körperteildaten, insbesondere als 3-dimensionalen Körperteildaten auch simulierte und durch ein Modell errechnete Daten verwendet werden. Diese basieren nicht auf tatsächlichen Daten des Inneren des aktuellen Körperteils, sondern sind beispielsweise auf der Basis von Datensammlungen von Körperteilen anderer Patienten simuliert und abgeschätzt worden.

Die Körperteildaten, insbesondere die 3-dimensionalen Körperteildaten können auch durch ein Vibrometrie-Verfahren erfasst werden. Ein derartiges Verfahren, bei dem mechanische Eigenschaften eines menschlichen Körperteils bestimmt werden, der insbesondere ein Amputationsstumpf ist, weist beispielsweise folgende Schritte auf:
a) der menschliche Körperteil wird zu einer mechanischen Schwingung angeregt,
b) die Schwingung wird durch eine Erfassungseinrichtung erfasst,
c) die erfasste Schwingung wird durch wenigstens eine elektronische Datenverarbeitungseinrichtung ausgewertet, die die wenigstens eine mechanische Eigenschaft des Körperteiles bestimmt.

Diesem Verfahren liegt die Erkenntnis zugrunde, dass eine Reihe mechanischer Eigenschaften, beispielsweise die Elastizität, Festigkeit und andere Schwingungseigenschaften ermittelt werden können, indem das jeweils zu untersuchende Körperteil zu einer mechanischen Schwingung angeregt wird, die erfasst und ausgewertet wird. Je nach Verteilung beispielsweise von Knochen und Weichteilen innerhalb eines Körperteils, beispielsweise eines Amputationsstumpfes, werden mechanische Schwingungen in unterschiedlichen Ausprägungen auftreten.

Die Erfassungseinrichtung ist in der Lage, die mechanische Schwingung zu erfassen. Dies geschieht durch Detektion und Bestimmung von Messwerten, die Aussagen über das Schwingungsverhalten des menschlichen Körperteiles enthalten. Dies können beispielsweise die Amplitude einer Schwingung, die Dämpfung und/oder die Frequenz einer Schwingung sein. Dabei ist es von Vorteil, die entsprechenden Messwerte nicht nur an einer Stelle des Körperteils, sondern bevorzugt über einen größeren Bereich, besonders bevorzugt über den gesamten Körperteil, aufzunehmen.

Um die so erfasste mechanische Schwingung auszuwerten und die mechanischen Eigenschaften des jeweiligen Körperteils zu bestimmen, verfügt die elektronische Datenverarbeitungseinrichtung vorzugsweise über ein theoretisches Modell, vorzugsweise ein 3-dimensionales Modell des Körperteils, um eine Verknüpfung zwischen den erfassten Messwerten und den gewünschten Eigenschaften herzustellen. Das Modell beinhaltet beispielsweise Annahmen über Lage, Orientierung, Größe und/oder Länge von Knochen, Muskeln oder sonstigen Organen und Weichteilen innerhalb des zu untersuchenden Körperteils. Besonders bevorzugt werden dem Modell Informationen zugrunde gelegt, die beispielsweise bereits vorhandenen MRT-Daten des Patienten, Geometrievorlagen oder sonstige Informationsquellen zu entnehmen sind. Dazu zählt beispielsweise der Indentor-Test. Insbesondere können 3-dimensionale Stumpfdaten verwendet werden, die durch andere Verfahren und/oder Untersuchungsmethoden und/oder Modelle und statistische Auswertungen generiert wurden.

Die erfassten Schwingungen, insbesondere die detektierten Messwerte, werden der wenigstens einen elektronischen Datenverarbeitungseinrichtung übermittelt. Diese verarbeitet die Daten und ermittelt beispielsweise Schwingungsdauern, Eigenmoden oder Dämpfungen, woraus dann beispielsweise die bewegten Massen und Steifigkeit errechnet werden können.

In einer bevorzugten Ausgestaltung wird der wenigstens eine Körperteil durch eine Anregungseinrichtung zu einer mechanischen Schwingung angeregt. Alternativ dazu kann der Körper auch selbst durch bewusste oder unbewusste Muskelkontraktionen in Schwingung versetzt werden. Eine reproduzierbare Anregung wird jedoch durch externe Anregungseinrichtung erreicht, die von außen auf den Körperteil einwirken.

Bevorzugt übt die Anregungseinrichtung wenigstens einen mechanischen Impuls, insbesondere einen Stoß, und/oder nächstens eine mechanische Schwingung, beispielsweise eine Vibration, auf den Körperteil aus. In besonders bevorzugten Ausgestaltungen ist die Anregungseinrichtung in der Lage, die Frequenz und/oder Amplitude einer mechanischen Schwingung, die auf den Körperteil ausgeübt wird, zu variieren, sodass diese Größen einstellbar und somit reproduzierbar sind. Die Anregungseinrichtung, die eingerichtet ist, mechanische Impulse, insbesondere Stöße, auf das Körperteil auszuüben, ist produktweise eingerichtet, den zeitlichen Abstand, die Stärke und/oder die Dauer der einzelnen mechanischen Impulse zu variieren, dass diese Größen einstellbar und damit reproduzierbar sind. Der mechanische Impuls kann in einem Stoß oder in einer anderen Bewegung von einem Teil des Gewebes des Körperteils bestehen. Während ein Stoß senkrecht oder zumindest nahezu senkrecht zur Haut des Körperteils erfolgt, kann beispielsweise auch eine Bewegung nahezu parallel zur Haut des Körperteils, die in einer Sperrung erfolgt, als mechanischer Impuls verwendet werden. Selbstverständlich sind auch Kombinationen der Bewegungsrichtungen möglich.

In einer bevorzugten Ausgestaltung des Verfahrens wird das menschliche Körperteil nacheinander in unterschiedliche mechanische Schwingungen versetzt, die vorzugsweise durch unterschiedliche Anregungen durch wenigstens eine Anregungseinrichtung erfolgen. Auf diese Weise können unterschiedliche Eigenmoden, unterschiedliche Schwingungen und unterschiedliche Reaktionen des wenigstens einen Körperteils auf die unterschiedlichen Anregungen erzeugt und studiert werden. Dabei können mehrere unterschiedliche Messwerte erzeugt und somit ein detaillierteres Modell verwendet werden. Insbesondere bei der Detektion und Untersuchung von Eigenmoden, die beispielsweise in Form von stehenden Schwingungswellen vorliegen können, insbesondere wenn die Anregungseinrichtung das wenigstens eine Körperteil in eine dauerhafte mechanische Schwingung versetzt, können auch Schwingungsknoten vorhanden sein, bei denen sich die Weichteile des jeweiligen Körperteils an dieser Stelle nicht oder nur minimal bewegen. Es ist daher von Vorteil, unterschiedliche Schwingungen des menschlichen Körperteils zu untersuchen, um auch an den Stellen, die bei einer ersten Schwingung in Schwingungsknoten angeordnet sind, Schwingungen hervorzurufen, die untersucht werden können.

Es ist daher von Vorteil, dass die Anregungseinrichtung des wenigstens einen Körperteils an wenigstens zwei unterschiedlichen Positionen und/oder zu mehreren Zeiten, mithin also mehrfach, anregt. Durch die Verwendung mehrerer Anregungspositionen können, wie bereits dargelegt, unterschiedliche Schwingungen erzeugt und beobachtet werden. Dadurch wird die Datenbasis, auf deren Grundlage die mechanischen Eigenschaften des Körperteils bestimmt werden, verbessert und vergrößert, sodass detailliertere Modelle verwendet werden können. Durch die mehrfache Anregung der gleichen Schwingung, die beispielsweise erzeugt wird, in dem ein Körperteil mehrfach identisch nacheinander an der gleichen Position angeregt wird, werden mehrfach Messungen der gleichen Schwingung durchgeführt, wodurch das Messergebnis und die Datenqualität verbessert wird.

Vorzugsweise beinhaltet die Erfassungseinrichtung wenigstens einen optischen Detektor, insbesondere wenigstens eine Kamera. Besonders bevorzugt verfügt die Erfassungseinrichtung über mehrere optische Detektoren, insbesondere mehrere Kameras, sodass die jeweilige Schwingung aus unterschiedlichen Richtungen erfasst wird. Auf diese Weise ist es beispielsweise möglich, den Amputationsstumpf vollständig, also insbesondere 3-dimensional, zu erfassen. Der wenigstens eine optische Detektor, insbesondere die wenigstens eine Kamera, ist auf das zu untersuchende Körperteil, bevorzugt also den Amputationsstumpf, gerichtet und detektiert die auftretenden Schwingungen. Über Bilderkennungs-Software können aus den so aufgenommenen Bildern unterschiedliche Schwingungsmoden, Frequenzen und/oder Amplituden detektiert und ausgelesen werden. Diese können in das Modell eingefügt werden, sodass die gewünschten mechanischen Eigenschaften berechnet werden können.

Besonders bevorzugt wird das wenigstens eine Körperteil mit einer Erfassungsstrahlung bestrahlt, deren Reflexion an dem Körperteil von der Erfassungseinrichtung detektiert wird. Die Erfassungsstrahlung ist vorzugsweise eine elektromagnetische Strahlung, besonders bevorzugt eine Laserstrahlung. Die Erfassungsstrahlung wird auf das zu beobachtende Körperteil geleitet und dort reflektiert. Insbesondere bei monochromatischer Laserstrahlung verändert diese aufgrund des Doppler-Effektes die Wellenlänge, wenn sie an einem sich bewegenden Objekt reflektiert wird. Bewegt sich der Punkt des Körperteils, an dem die Laserstrahlung auftrifft, im Moment des Auftreffens gerade auf den optischen Detektor zu, wird die Frequenz der Laserstrahlung erhöht. Bewegt sich die Stelle vom optischen Detektor weg, wird die Frequenz gesenkt. Auf diese Weise kann eine Abbildung des Amputationsstumpfes oder des wenigstens einen Körperteils erzeugt werden, wobei jeweils die Frequenz der reflektierten Laserstrahlung als Funktion des Auftreffpunktes dargestellt wird. Auf diese Weise lässt sich ein Geschwindigkeitsbild, also eine Verteilung der jeweiligen Geschwindigkeit und damit der Schwingung, darstellen.

In einer bevorzugten Ausgestaltung ist das wenigstens eine Körperteil mit einer Markierung versehen. Vorzugsweise ist die Form, Größe, Richtung und Anordnung der Markierung in einem elektronischen Datenspeicher der wenigstens einen elektronischen Datenverarbeitungseinrichtung hinterlegt. Es ist folglich bekannt, wie die Markierung aussieht, in der wenigstens ein Körperteil nicht in Schwingung versetzt wird. Wird der Körperteil zur Schwingung angeregt, gerät zumindest die Haut, auf der sich die Markierung befindet, in Bewegung, sodass sich bevorzugt auch die Markierung bewegt. Da die Haut jedoch nicht homogen bewegt wird, wenn der wenigstens eine Körperteil in Schwingung versetzt wird, kommt es bei der Markierung zu Verschiebungen, Verzerrungen und Umformungen, die detektiert oder ausgewertet werden können.

Vorzugsweise ist die wenigstens eine Markierung auf das Körperteil aufgebracht, insbesondere auf ihn aufgeklebt, aufgesprüht oder in Form einer Beschichtung oder Beschriftung aufgebracht. Danach wird zunächst das Körperteil mit der aufgebrachten Markierung vermessen, also beispielsweise durch die Erfassungseinrichtung erfasst, ohne dass er in Schwingungen versetzt wurde. Alternativ oder zusätzlich dazu kann auch eine Markierung auf das Körperteil projiziert werden, indem er beispielsweise einer streifenförmigen Beleuchtung ausgesetzt wird. Dies bedeutet, das Körperteil wird nicht vollflächig beleuchtet, sondern mit einem Muster aus hellen und dunklen Bereichen beleuchtet, das ebenfalls von der wenigstens einen Erfassungseinrichtung detektiert werden kann. Auch wenn sich die Markierung, also die Beleuchtung selbst, durch die Schwingungen nicht verändert, verändert sich doch die Position, Lage und Orientierung einzelner Hautabschnitte des wenigstens einen Körperteiles relativ zur Lichtquelle, sodass sich der Körperteil relativ zur Markierung bewegt. Auch dies führt zu einer Veränderung der Anordnung der beleuchteten und nicht beleuchteten Bereiche auf dem Körperteil, die detektiert und ausgewertet werden können.

Die mechanische Eigenschaft, die durch die hier beschriebenen Verfahren detektiert wird, beinhaltet vorzugsweise die Lage und/oder die Position wenigstens eines inneren Gewebe- und/oder Organbereiches, einer Materialzusammensetzung, eine Elastizität und/oder eine Schwingungsdämpfung. Besonders bevorzugt handelt es sich bei dem menschlichen Körperteil um einen Amputationsstumpf.

Vorzugsweise werden die 3-dimensionalen Produktdaten mittels der Anzeigeeinrichtung dargestellt. Besonders bevorzugt werden sie den Körperteildaten überlagert dargestellt.

Bevorzugt beinhalten die Körperteildaten, insbesondere die 3-dimensionalen Körperteildaten Informationen über Knochen, Muskeln, Nerven, Blutgefäße und/oder Weichteile des Köperteils. Dies betrifft die Lage, Orientierung und Verlauf der entsprechenden Teile.

Erfindungsgemäß wird vor dem Erzeugen der Produktionsdaten das Körperteil verformt und/oder abgeformt, wobei dann entsprechend geänderte Bilddaten mittels der Anzeigeeinrichtung dargestellt werden. Die Verformung des Körperteils kann beispielsweise per Hand durch den Orthopädietechniker erfolgen. Die bei bisherigen Verfahren, bei denen ein Abformen durch Gips verwendet wird, gemachten Erfahrungen können so auch bei dem neuen Verfahren verwendet werden. Der Orthopädietechniker bringt das Körperteil in die gewünschte Form. Wird eine Kameraeinrichtung verwendet, erfasst diese die Bilddaten. Diese Bilddaten, die entsprechend der vorgenommenen Verformung des Körperteils geändert sind, werden mittels der Anzeigeeinrichtung dargestellt. Auf diese Weise können auch virtuell dargestellte und vorkonstruierte orthopädietechnische Produkte an die geänderten Abmessungen und Formen des jeweiligen Körperteils angepasst werden.

Vorzugsweise berechnet eine elektronische Datenverarbeitungseinrichtung mittels eines zugrunde liegenden Modelles welche Auswirkungen die Verformung und/oder Abformung des Körperteils auf den inneren Aufbau des Körperteils hat. Dies betrifft beispielsweise die Verschiebung oder Bewegung von Knochen und Muskeln, bei denen sich durch die Verformung und/oder Abformung des Körperteils der Verlauf und/oder die Länge ändern kann. Weichteile können durch die Verformung des Körperteils gestaucht, in der Form verändert oder verschoben werden. Alle diese Änderungen, die durch die Verformung des Körperteils auftreten, werden in der bevorzugten Ausgestaltung durch die elektronische Datenverarbeitungseinrichtung berechnet. Auf der Grundlage des Ergebnisses dieser Berechnung werden die Körperteildaten modifiziert und in dieser modifizierten Form auf der Anzeigeeinrichtung dargestellt. Dort werden nun die modifizierten Körperteildaten den geänderten Bilddaten überlagert dargestellt, sodass der Orthopädietechniker den Effekt und das Resultat der von ihm vorgenommenen Verformung und/oder Abformung des Körperteils unmittelbar in Augenschein nehmen kann. Er ist dabei nicht mehr nur auf seine Erfahrung und sein Gefühl reduziert, sondern kann prüfen, ob die von ihm vorgenommene Verformung und/oder Abformung des Körperteils den gewünschten Effekt aufweist. Ist dies nicht der Fall, kann er korrigieren und eine andere Verformung und/oder Abformung des Körperteils vornehmen. Vorzugsweise werden auf der Grundlage der modifizierten Körperteildaten und/oder der geänderten Bilddaten auch modifizierte 3-dimensionale Produktdaten erzeugt, auf deren Grundlage die Produktionsdaten erzeugt werden.

Wie bereits dargelegt, wird die Verformung und/oder Abformung des Körperteils mittels wenigstens eines Scanhandschuh und/oder eines Scanliner erfasst.

Bevorzugt wird nach dem Erzeugen der Produktionsdaten simuliert, welche Auswirkungen eine Belastung und/oder eine Bewegung des Körperteiles im angelegten Zustand des orthopädietechnischen Produktes auf das Körperteil hat und das Ergebnis dieser Simulation mittels der Anzeigeeinrichtung angezeigt. Auf diese Weise wird überprüft, ob die Produktionsdaten zu einem orthopädietechnischen Produkt führen, das den Anforderungen gerecht wird und einen ausreichenden Tragekomfort bei gleichzeitiger Funktionsfähigkeit gewährleistet. Dies geschieht bevorzugt mittels der elektronischen Datenverarbeitungseinrichtung, die ein Modell verwendet, um auftretende Belastungen und/oder mögliche Bewegungen des Körperteils zu simulieren. Dabei wird ermittelt, wo beispielsweise bei bestimmter Belastung und/oder bei bestimmten Bewegungen besonders hohe Drücke oder Reibungen auftreten, die zu Schmerzen und Wunden führen können, zumindest aber den Tragekomfort deutlich reduzieren.

Die so errechneten Daten geben Rückschlüsse beispielsweise auf die Belastungen innerhalb eines Gangzyklus. Der Benutzer des Verfahrens, insbesondere ein Orthopädietechniker ist dann in der Lage, Änderungen an den Produktdaten und den Produktionsdaten vorzunehmen und diese so an die dynamischen Gegebenheiten anzupassen. Im Idealfall ist dadurch die Fertigung eines Testproduktes, beispielsweise eines Testschaftes für die Überprüfung unter Last nicht mehr notwendig und es kann direkt das endgültige Produkt gefertigt werden.

Zusätzlich oder alternativ dazu können Bewegungen des Körperteils, insbesondere eines Amputationsstumpfes, mittels der Kameraeinrichtung und/oder einer Scan-Einrichtung erfasst werden. Diese Bewegungen können dann simuliert werden, sodass mittels der elektronischen Datenverarbeitungseinrichtung errechnet werden kann, wie sich diese tatsächlich durchgeführten und durchführbaren Bewegungen auf die Körperteildaten auswirken, insbesondere dann, wenn das orthopädietechnische Produkt gemäß den bereits erstellten Produktionsdaten hergestellt und getragen würde. Dies beinhaltet Belastungen und Bewegungen, die insbesondere dann wichtig sind, wenn das orthopädietechnische Produkt ein Gelenk des Trägers betrifft.

Vorzugsweise ist die Blickrichtung veränderbar. Dies ist insbesondere dann von Vorteil, wenn beispielsweise einzelne Elemente des inneren Aufbaus des Körperteils nur in unterschiedlichen Blickrichtungen erkennbar sind. Alternativ oder zusätzlich ist die Veränderung der Blickrichtung von Vorteil, um den herzustellenden Prothesenschaft in möglichst jeder Richtung optimal an das Körperteil anzupassen. Auch können Veränderungen der Form des Körperteils aus unterschiedlichen Blickrichtungen gesehen unterschiedliche Änderungen des inneren Aufbaus hervorrufen und so unterschiedliche Modifizierungen der Körperteildaten nötig machen.

In einer bevorzugten Ausgestaltung des Verfahrens wird die Blickrichtung aus einer Lage und Orientierung des Körperteils relativ zur Kameraeinrichtung, insbesondere einer Kamera, berechnet. Dies kann auf unterschiedliche Weise geschehen. Ist beispielsweise die Position und Orientierung der wenigstens einen Kamera, die die Kameraeinrichtung aufweist, bekannt, kann diese Position und Orientierung zur Grundlage der Berechnung gemacht werden. Ist zusätzlich auch die Position und Orientierung beispielsweise einer Halterung oder einer Auflage bekannt, in der das Körperteil gehalten oder auf der er aufgelegt ist, können diese beiden Positionen und Orientierungen verwendet werden, um die Blickrichtung zu berechnen.

Besonders bevorzugt wird die Lage und Orientierung des Körperteils relativ zu der Kameraeinrichtung aus den erfassten Bilddaten errechnet, wobei das Körperteil vorzugsweise wenigstens eine Markierung, insbesondere einen Aufkleber und/oder eine Beschriftung, aufweist. Die Kameraeinrichtung verfügt über wenigstens eine Kamera, die das Körperteil erfasst und die Bilddaten aufnimmt. Ist die Form des Körperteils bekannt, kann beispielsweise über eine Bilderkennungssoftware in der elektronische Datenverarbeitungseinrichtung errechnet werden, in welcher Orientierung und Lage des Körperteils zur Kamera angeordnet ist und wie weit er von ihr entfernt ist. Damit sind alle Daten bekannt, um die Blickrichtung zu bestimmen. Alternativ oder zusätzlich dazu kann auch wenigstens eine Markierung am Körperteil angeordnet werden. Geschieht dies an vorher definierten Stellen ist es möglich, aus den erkannten Bilddaten die Position und Orientierung der Markierungen, beispielsweise wenigstens eines Aufklebers, zu bestimmen und so aus der bekannten Position der Markierungen am Körperteil die Position und Orientierung des Körperteils relativ zur Kamera zu bestimmen. Auch dadurch kann die Blickrichtung eindeutig festgelegt werden.

Die eigentliche Anpassung des Produktes an das Körperteil geschieht vorzugsweise bis zum Erzeugen der Produktionsdaten. Diese können dann einer herkömmlichen Produktionsstätte übermittelt werden, die nicht notwendigerweise identisch ist mit dem Ort, an dem die bisherigen Verfahrensschritte durchgeführt werden. So ist es beispielsweise möglich, Daten elektronisch, insbesondere kabellos, über Netzwerke, beispielsweise das Internet, der Produktionsstätte zu übermitteln, die an nahezu jedem Ort auf der Welt angesiedelt sein kann.

Die Erfindung löst die gestellte Aufgabe zudem durch ein Verfahren zum Darstellen von Körperteildaten, insbesondere von 3-dimensionalen Körperteildaten, das in einem der hier beschriebenen Verfahren verwendbar ist. Dabei werden die Körperteildaten derart dargestellt, dass ein Benutzer des Verfahrens sowohl die Körperteildaten als auch das Körperteil selbst überlagert sieht. Dabei sieht er sowohl das Körperteil als auch die Körperteildaten aus der aktuellen Blickrichtung.

Mit einem derartigen Verfahren kann beispielsweise ein Orthopädietechniker eine bereits hergestellte Orthese an die individuellen Gegebenheiten und Bedürfnisse des Patienten anpassen und beispielsweise Gelenkachsen, Gelenkpositionen und/oder Gelenksorientierungen anpassen, einstellen oder verschieben. Auch bei diesem Verfahren ist es von Vorteil, wenn Änderungen, die beispielsweise an der Orthese und/oder dem Körperteil vorgenommen werden und die Einfluss auf das Innere des Körperteils haben, von der elektronischen Datenverarbeitungseinrichtung erfasst und verarbeitet werden. Die elektronische Datenverarbeitungseinrichtung ist daher bevorzugt in der Lage, den Einfluss dieser Änderungen zu modellieren und die Körperteildaten entsprechend anzupassen und die so angepassten Körperteildaten anzuzeigen.

Die Erfindung löst die gestellte Aufgabe zudem durch eine Vorrichtung zum Durchführen eines hier beschriebenen Verfahrens, wobei die Vorrichtung wenigstens eine Kameraeinrichtung, wenigstens eine Anzeigeeinrichtung und wenigstens eine elektronische Datenverarbeitungseinrichtung aufweist.

In einer besonders bevorzugten Ausgestaltung sind die Kameraeinrichtung und die Anzeigeeinrichtung Teile einer einzigen Einrichtung, die an einem Kopf des Benutzers anzuordnen ist. Es handelt sich beispielsweise um eine Brille, insbesondere eine VR-Brille (VR: Virtual Reality), eine AR-Brille (AR: Augmented Reality) oder eine entsprechende Haube oder einen Helm. Mit einer solchen Einrichtung ist es für den Benutzer der Vorrichtung, der das Verfahren durchführen möchte, besonders einfach möglich, den Amputationsstumpf aus unterschiedlichen Blickwinkeln zu betrachten und den Prothesenschaft entsprechend auszubilden.

Mithilfe der beigefügten Zeichnungen werden nachfolgend einige Ausführungsbeispiele der vorliegenden Erfindung näher erläutert. Es zeigen
- Figur 1 und 2: - schematische Darstellungen von Verfahrensabläufen und
- Figur 3: - die schematische Darstellung von Bilddaten und 3-dimensionalen Körperteildaten.

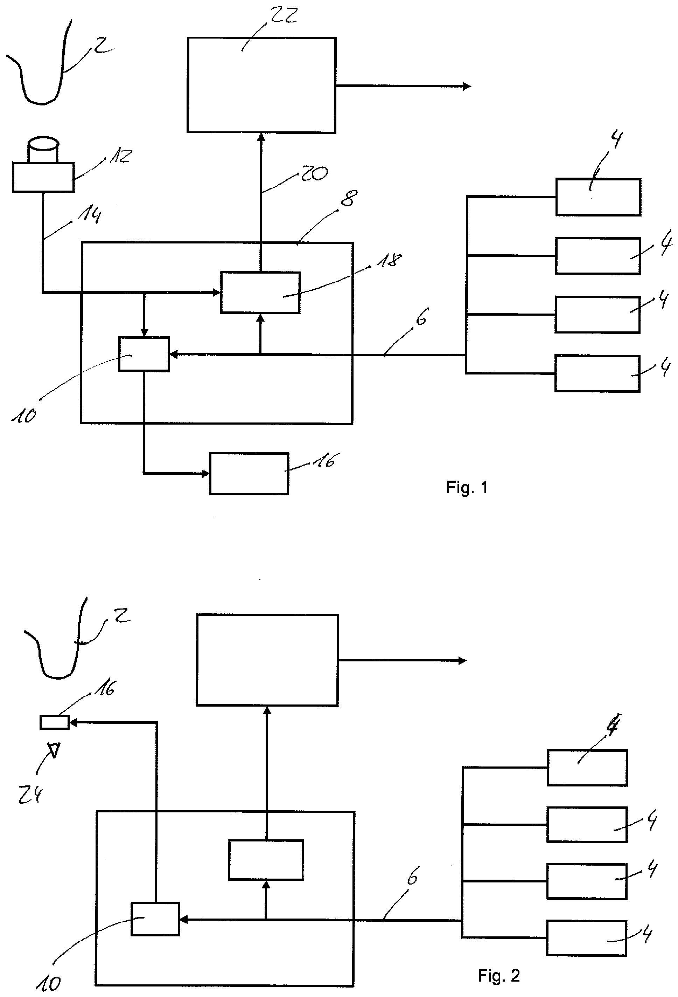

Figur 1 zeigt schematisch einen Aufbau und Verfahrensablauf, wie er für ein Ausführungsbeispiel der vorliegenden Erfindung verwendet werden kann. Für ein schematisch dargestelltes Körperteil 2, das als Amputationsstumpf dargestellt ist, soll ein orthopädietechnisches Produkt erstellt werden. Dazu werden zunächst beispielsweise über medizinische Verfahren 4 Körperteildaten 6 bereitgestellt. Dabei können unterschiedlichste medizinische Verfahren 4 verwendet werden. Dargestellt sind unterschiedliche medizinische Verfahren 4, die jeweils schematisch dargestellt sind. selbstverständlich können auch mehrere oder weniger medizinische Verfahren 4 verwendet werden, um Körperteildaten 6 bereitzustellen.

Körperteildaten 6 werden einer elektronischen Datenverarbeitungseinrichtung 8 bereitgestellt, die über ein Darstellungsmodul 10 verfügt. Diesem Darstellungsmodul werden die Körperteildaten 6 übermittelt. Das Körperteil 2 wird zudem von einer Kameraeinrichtung 12 erfasst. Diese sendet Bilddaten 14 an die elektronische Datenverarbeitungseinrichtung 8 und darin insbesondere an das Darstellungsmodul 10. Die Kameraeinrichtung 12 erfasst das Körperteil 2 in der aktuellen Blickrichtung. Die Kameraeinrichtung 12 kann dabei auf sichtbarem Licht, UV-Licht oder Infrarot-Wellen basieren und entsprechende Bilder des Körperteils 2 aufnehmen.

Das Darstellungsmodul 10 der elektronischen Datenverarbeitungseinrichtung 8 ist eingerichtet, aus den ihm zur Verfügung gestellten Körperteildaten 6 und den Bilddaten 14 ein gemeinsames Bild zu machen und diese überlagert darzustellen. Dazu verfügt die Vorrichtung über eine Anzeigeeinrichtung 16, die beispielsweise ein Monitor ist. Das Darstellungsmodul 10 erkennt oder kennt die aktuelle Blickrichtung und stellt auch die ihm übermittelten Körperteildaten 6 aus der aktuellen Blickrichtung dar, sodass ein nicht dargestellter Benutzer des Verfahrens auf der Anzeigeeinrichtung 16 die Körperteildaten 6 und die Bilddaten 14 überlagert aus der gleichen aktuellen Blickrichtung angezeigt bekommt.

Die Körperteildaten 6 und die Bilddaten 14 werden zudem einem Erstellungsmodul 18 der elektronischen Datenverarbeitungseinrichtung 8 übermittelt. Dieses erstellt auf der Grundlage der übermittelten Daten Produktionsdaten 20, die einer Produktionsstätte 22 übermittelt werden. Dies kann beispielsweise ein 3-D-Drucker oder eine sonstige Fertigungseinrichtung sein.

Figur 2 zeigt eine weitere Ausgestaltung, die sich nur in einem Detail von der in Figur 1 gezeigten Ausgestaltung unterscheidet. Das Körperteil 2 wird nicht von einer Kameraeinrichtung 12, sondern vom Benutzer 24 des Verfahrens, der schematisch als Auge dargestellt ist, beobachtet. Dabei blickt er durch die Anzeigeeinrichtung 16, die beispielsweise eine AR-Brille ist. Die Anzeigeeinrichtung 16 ist teiltransparent ausgebildet, sodass der Benutzer 24 sowohl das Körperteil 2 als auch die vom Darstellungsmodul 10 bereitgestellten Daten sieht. Bei diesen handelt es sich jedoch ausschließlich um Körperteildaten, insbesondere um 3-dimensionale Körperteildaten 6, die von den unterschiedlichen medizinischen Verfahren 4 ermittelt und bereitgestellt wurden. Sowohl beim Ablauf gemäß Figur 1 als auch beim Ablauf gemäß Figur 2 werden die Körperteildaten 6 selbstverständlich nicht direkt von den medizinischen Verfahren 4 bereitgestellt. Vielmehr werden sie von den medizinischen Verfahren 4 erstellt und in einem elektronischen Datenspeicher, der nicht dargestellt ist, hinterlegt, auf den die elektronische Datenverarbeitungseinrichtung 8 zugreifen kann.

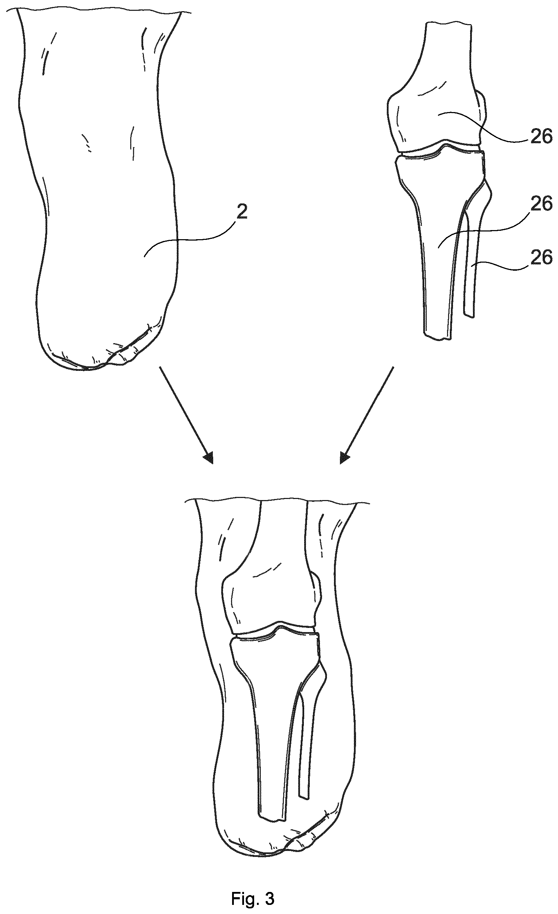

Figur 3 zeigt schematisch die unterschiedlichen Daten, die überlagert dargestellt werden. Im linken oberen Bereich der Figur 3 sind Bilddaten 14 eines Amputationsstumpfes, also eines Körperteils 2 dargestellt. Im rechten oberen Bereich sind die Knochen 26, die sich im Innern des Körperteils 2 befinden, dargestellt. Diese Daten sind beispielsweise durch ein MRT-Verfahren erzeugt worden und sind in einem elektronischen Datenspeicher hinterlegt. Es handelt sich folglich um 3-dimensionale Körperteildaten 6. Im unteren Bereich der Figur 3 sind die Bilddaten 14 und die 3-dimensionalen Körperteildaten 6 überlagert dargestellt, wie dies auf der Anzeigeeinrichtung 16 beispielsweise bei der in Figur 1 gezeigten Anordnung der Fall wäre.

### Bezugszeichenliste

- 2: Körperteil
- 4: medizinisches Verfahren
- 6: Körperteildaten
- 8: elektronische Datenverarbeitungseinrichtung
- 10: Darstellungsmodul
- 12: Kameraeinrichtung
- 14: Bilddaten
- 16: Anzeigeeinrichtung
- 18: Erstellungsmodul
- 20: Produktionsdaten
- 22: Produktionsstätte
- 24: Benutzer
- 26: Knochen

## Patentansprüche

1. Verfahren zum Herstellen eines orthopädietechnischen Produktes für ein Körperteil eines Patienten, wobei das Verfahren folgende Schritte aufweist:
a) Bereitstellen von Körperteildaten, die Informationen über einen inneren Aufbau des Körperteils enthalten,
b) Erfassen einer aktuellen Blickrichtung, aus der ein Benutzer des Verfahrens das Körperteil sieht,
c) Darstellen der Körperteildaten aus der aktuellen Blickrichtung mittels der Anzeigeeinrichtung, so dass der Benutzer das Körperteil und die Körperteildaten überlagert sieht,
d) Verformen und/oder Abformen des Körperteils,
e) Erfassen von Bilddaten des verformten und/oder abgeformten Körperteils mittels einer Kamera und/oder einer Scanvorrichtung zumindest auch aus der aktuellen Blickrichtung, die mittels der Anzeigeeinrichtung den Körperteildaten überlagert dargestellt werden,
f) Erzeugen von Produktionsdaten für die orthopädietechnische Einrichtung auf der Grundlage der dargestellten Daten und
g) Bereitstellen eines auf der Grundlage der Produktionsdaten hergestellten orthopädietechnischen Produktes.

2. Verfahren nach Anspruch 1, **dadurch gekennzeichnet, dass** die Körperteildaten 3-dimensionale Körperteildaten sind.

3. Verfahren nach Anspruch 1 oder 2, **dadurch gekennzeichnet, dass** die Anzeigeeinrichtung teiltransparent ist, so dass der Benutzer durch die Anzeigeeinrichtung hindurch blicken kann.

4. Verfahren nach einem der vorstehenden Ansprüche, **dadurch gekennzeichnet, dass** zum Erzeugen der Produktionsdaten zunächst 3-dimensionale Produktdaten auf der Grundlage der Körperteildaten und/oder der erfassten Bilddaten erzeugt werden, auf deren Grundlage die Produktionsdaten erzeugt werden.

5. Verfahren nach einem der vorstehenden Ansprüche, **dadurch gekennzeichnet, dass** die Körperteildaten durch ein bildgebendes medizinisches Verfahren gewonnen wurden oder im Rahmen des Verfahrens vor dem Bereitstellen gewonnen werden.

6. Verfahren nach Anspruch 4 oder 5, **dadurch gekennzeichnet, dass** die 3-dimensionalen Produktdaten mittels der Anzeigeeinrichtung dargestellt werden, wobei sie bevorzugt den Körperteildaten überlagert dargestellt werden.

7. Verfahren nach Anspruch einem der vorstehenden Ansprüche, **dadurch gekennzeichnet, dass** die Körperteildaten Informationen über Knochen, Muskeln, Nerven, Blutgefäße und/oder Weichteile des Körperteiles enthalten.

8. Verfahren nach einem der vorstehenden Ansprüche, **dadurch gekennzeichnet, dass** vor dem Erzeugen der Produktionsdaten das Körperteil verformt und/oder abgeformt wird und eine elektronische Datenverarbeitungseinrichtung mittels eines Modells berechnet, welche Auswirkungen die Verformung und/oder Abformung des Körperteiles auf den inneren Aufbau des Körperteils hat und die Körperteildaten entsprechend modifiziert, wobei dann die modifizierten Körperteildaten dargestellt werden.

9. Verfahren nach Anspruch 8, **dadurch gekennzeichnet, dass** zum Erzeugen der Produktionsdaten an die vorgenommene Verformung und/oder Abformung angepasste 3-dimensionale Produktdaten erzeugt werden.

10. Verfahren nach Anspruch 8 oder 9, **dadurch gekennzeichnet, dass** die Verformung und/oder Abformung des Körperteiles mittels wenigstens eines Scanhandschuhs und/oder eines Scanliners erfasst wird.

11. Verfahren nach einem der vorstehenden Ansprüche, **dadurch gekennzeichnet, dass** nach dem Erzeugen der Produktionsdaten simuliert wird, welche Auswirkungen eine Belastung und/oder eine Bewegung des Körperteiles im angelegten Zustand des orthopädietechnischen Produktes auf das Körperteil hat und das Ergebnis dieser Simulation mittels der Anzeigeeinrichtung angezeigt wird.

12. Verfahren nach einem der vorstehenden Ansprüche, **dadurch gekennzeichnet, dass** die Blickrichtung veränderbar ist.

13. Verfahren nach einem der vorstehenden Ansprüche, **dadurch gekennzeichnet, dass** die Blickrichtung aus einer Lage und Orientierung des Köperteiles relativ zu der Kameraeinrichtung, insbesondere einer Kamera berechnet wird.

14. Verfahren nach Anspruch 13, **dadurch gekennzeichnet, dass** die Lage und Orientierung des Körperteils relativ zu der Kameraeinrichtung aus den erfassten Bilddaten errechnet wird, wobei das Körperteil vorzugsweise wenigstens eine Markierung, insbesondere einen Aufkleber und/oder eine Beschriftung aufweist.

15. Verfahren zum Erzeugen von Produktionsdaten für ein orthopädietechnisches Produkt, wobei das Verfahren folgende Schritte aufweist:
a) Bereitstellen von Körperteildaten, die Informationen über einen inneren Aufbau des Körperteils enthalten,
b) Erfassen einer aktuellen Blickrichtung, aus der ein Benutzer des Verfahrens das Körperteil sieht,
c) Darstellen der Körperteildaten aus der aktuellen Blickrichtung mittels der Anzeigeeinrichtung, so dass der Benutzer das Körperteil und die Körperteildaten überlagert sieht,
d) Verformen und/oder Abformen des Körperteils,
e) Erfassen von Bilddaten des verformten und/oder abgeformten Körperteils mittels einer Kamera und/oder einer Scanvorrichtung zumindest auch aus der aktuellen Blickrichtung, die mittels der Anzeigeeinrichtung den Körperteildaten überlagert dargestellt werden,
f) Erzeugen von Produktionsdaten für die orthopädietechnische Einrichtung auf der Grundlage der dargestellten Daten.

16. Vorrichtung eingerichtet zum Durchführen eines Verfahrens nach einem der vorstehenden Ansprüche, wobei die Vorrichtung wenigstens eine Kameraeinrichtung,
wenigstens eine Anzeigeeinrichtung und wenigstens eine elektronische Datenverarbeitungseinrichtung aufweist.

17. Vorrichtung nach Anspruch 16, **dadurch gekennzeichnet, dass** die Kameraeinrichtung und die Anzeigeeinrichtung Teile eines am Kopf eines Benutzers anzuordnenden Einrichtung, insbesondere einer Brille, einer Haube oder eines Helmes sind.

## Claims

1. A method for producing an orthopedic product for a body part of a patient, the method comprising the following steps:
a) providing body part data that contains information about the inner structure of the body part,
b) detecting a current direction of view from which a user of the method sees the body part,
c) presenting the body part data from the current direction of view by means of the display device, so that the user sees the body part and the body part data superimposed,
d) deforming and/or molding the body part,
e) capturing image data of the deformed and/or molded body part by means of a camera and/or a scan device at least also from the current direction of view, said image data being displayed superimposed on the body part data by means of the display device,
f) generating production data for the orthopedic product on the basis of the data displayed, and
g) providing an orthopedic product produced on the basis of the production data.

2. The method according to claim 1, **characterized in that** the body part data is three-dimensional body part data.

3. The method according to claim 1 or 2, **characterized in that** the display device is partially transparent, so that the user can look through the display device.

4. The method according to one of the preceding claims, **characterized in that**, in order to generate the production data three-dimensional product data is initially generated on the basis of the body part data and/or the captured image data, on the basis of which the production data is generated.

5. The method according to one of the preceding claims, **characterized in that** the body part data has been obtained by a medical imaging process or is obtained as part of the method prior to its provision.

6. The method according to claim 4 or 5, **characterized in that** the three-dimensional product data is displayed using the display device, wherein it is preferably shown superimposed on the body part data.

7. The method according to one of the preceding claims, **characterized in that** the body part data contains information on bones, muscles, nerves, blood vessels and/or soft tissues of the body part.

8. The method according to one of the preceding claims, **characterized in that** prior to generating the production data, the body part is preferably deformed and/or molded and an electronic data processing device uses a model to calculate the effects of the deformation and/or molding of the body part on the internal structure of the body part and modifies the body part data accordingly, wherein the modified body part data is then displayed.

9. The method according to claim 8, **characterized in that** three-dimensional product data adapted to the deformation and/or molding is generated in order to generate the production data.

10. The method according to claim 8 or 9, **characterized in that** the deformation and/or molding of the body part is captured by means of at least one scanning glove and/or scan liner.

11. The method according to one of the preceding claims, **characterized in that**, after generating the production data, the effects of a load and/or a movement of the body part when the orthopedic product is in the mounted state on the body part are simulated and the result of this simulation is displayed by means of the display device.

12. The method according to one of the preceding claims, **characterized in that** the direction of view can be changed.

13. The method according to one of the preceding claims, **characterized in that** direction of view is calculated from a position and orientation of the body part relative to the camera device, in particular a camera.

14. The method according to claim 13, **characterized in that** the position and orientation of the body part relative to the camera device is calculated from the captured image data, the body part preferably featuring at least one marker, particularly a sticker and/or label.

15. A method for generating production data for an orthopedic product, wherein the method comprises the following steps:
a) providing body part data that contains information about the inner structure of the body part,
b) detecting a current direction of view from which a user of the method sees the body part,
c) presenting the body part data from the current direction of view by means of the display device, so that the user sees the body part and the body part data superimposed,
d) deforming and/or molding the body part,
e) capturing image data of the deformed and/or molded body part by means of a camera and/or a scan device at least also from the current direction of view, said image data being displayed superimposed on the body part data by means of the display device,
f) generating production data for the orthopedic product on the basis of the data displayed.

16. A device for conducting a method according to one of the preceding claims, the device comprising at least one camera device, at least one display device and at least one electronic data processing device.

17. The method according to claim 16, **characterized in that** the camera device and the display device form part of a device to be arranged on the head of a user, especially glasses/goggles, a hood or a helmet.

## Revendications

1. Procédé de fabrication d'un produit orthopédique pour une partie du corps d'un patient, le procédé comprenant les étapes suivantes consistant à :
a) fournir des données relatives à la partie du corps, qui contiennent des informations sur la structure interne de la partie du corps,
b) saisir une direction de regard actuelle depuis laquelle un utilisateur du procédé voit la partie du corps,
c) représenter les données relatives à la partie du corps depuis la direction de regard actuelle à l'aide d'une unité d'affichage, de sorte que l'utilisateur voie de manière superposée la partie du corps et les données relatives à la partie du corps,
d) déformer et/ou mouler la partie du corps,
e) enregistrer des données d'image de la partie du corps déformée et/ou moulée à l'aide d'une caméra et/ou d'un dispositif de balayage, au moins également depuis la direction de regard actuelle, qui sont représentées de manière superposée aux données relatives à la partie du corps à l'aide de l'unité d'affichage,
f) générer des données de production pour le dispositif orthopédique sur la base des données représentées, et
g) fournir un produit orthopédique fabriqué sur la base des données de production.

2. Procédé selon la revendication 1,
**caractérisé en ce que** les données relatives à la partie du corps sont des données tridimensionnelles relatives à la partie du corps.

3. Procédé selon la revendication 1 ou 2,
**caractérisé en ce que** l'unité d'affichage est partiellement transparente, de manière à permettre à l'utilisateur de voir à travers l'unité d'affichage.

4. Procédé selon l'une des revendications précédentes,
**caractérisé en ce que**, pour générer les données de production, tout d'abord des données de produit tridimensionnelles sont générées sur la base des données relatives à la partie du corps et/ou des données d'image enregistrées, sur la base desquelles les données de production sont générées.

5. Procédé selon l'une des revendications précédentes,
**caractérisé en ce que** les données relatives à la partie du corps ont été obtenues par un procédé médical d'imagerie ou sont obtenues dans le cadre du procédé avant de les fournir.

6. Procédé selon la revendication 4 ou 5,
**caractérisé en ce que** les données de produit tridimensionnelles sont représentées à l'aide de l'unité d'affichage, de préférence elles sont représentées de manière superposée aux données relatives à la partie du corps.

7. Procédé selon l'une des revendications précédentes,
**caractérisé en ce que** les données relatives à la partie du corps contiennent des informations sur les os, les muscles, les nerfs, les vaisseaux sanguins et/ou les tissus mous de la partie du corps.

8. Procédé selon l'une des revendications précédentes,
**caractérisé en ce que**, avant de générer les données de production, la partie du corps est déformée et/ou moulée, et une unité de traitement électronique des données calcule, à l'aide d'un modèle, les effets de la déformation et/ou du moulage de la partie du corps sur la structure interne de la partie du corps et modifie en correspondance les données relatives à la partie du corps, les données modifiées relatives à la partie du corps étant alors représentées.

9. Procédé selon la revendication 8,
**caractérisé en ce que**, pour générer les données de production, des données de produit tridimensionnelles adaptées à la déformation et/ou au moulage effectué(e) sont générées.

10. Procédé selon la revendication 8 ou 9,
**caractérisé en ce que** la déformation et/ou le moulage de la partie du corps est enregistré(e) à l'aide d'au moins un gant de balayage et/ou d'une doublure de balayage.

11. Procédé selon l'une des revendications précédentes,
**caractérisé en ce qu'**après la génération des données de production, il est prévu de simuler les effets d'une contrainte et/ou d'un mouvement de la partie du corps, à l'état appliqué du produit orthopédique, sur la partie du corps et le résultat de cette simulation est affiché à l'aide de l'unité d'affichage.

12. Procédé selon l'une des revendications précédentes,
**caractérisé en ce que** la direction du regard est modifiable.

13. Procédé selon l'une des revendications précédentes,
**caractérisé en ce que** la direction du regard est calculée à partir d'une position et d'une orientation de la partie du corps par rapport à l'unité de caméra, en particulier par rapport à une caméra.

14. Procédé selon la revendication 13,
**caractérisé en ce que** la position et l'orientation de la partie du corps par rapport à l'unité de caméra sont calculées à partir des données d'image enregistrées, la partie du corps présentant de préférence au moins un marquage, en particulier un autocollant et/ou une inscription.

15. Procédé de génération de données de production pour un produit orthopédique, le procédé comprenant les étapes suivantes consistant à :
a) fournir des données relatives à une partie du corps, qui contiennent des informations sur la structure interne de la partie du corps,
b) saisir une direction de regard actuelle depuis laquelle un utilisateur du procédé voit la partie du corps,
c) représenter les données relatives à la partie du corps depuis la direction de regard actuelle à l'aide de l'unité d'affichage, de sorte que l'utilisateur voie de manière superposée la partie du corps et les données relatives à la partie du corps,
d) déformer et/ou mouler la partie du corps,
e) enregistrer des données d'image de la partie du corps déformée et/ou moulée à l'aide d'une caméra et/ou d'un dispositif de balayage, au moins également depuis la direction de regard actuelle, qui sont représentées de manière superposée aux données relatives à la partie du corps à l'aide de l'unité d'affichage,
f) générer des données de production pour le dispositif orthopédique sur la base des données représentées.

16. Dispositif conçu pour mettre en œuvre un procédé selon l'une des revendications précédentes, le dispositif comportant au moins une unité de caméra, au moins une unité d'affichage et au moins une unité électronique de traitement de données.

17. Dispositif selon la revendication 16,
**caractérisé en ce que** l'unité de caméra et l'unité d'affichage sont des parties d'une unité à placer sur la tête d'un utilisateur, en particulier des lunettes, une coiffe ou un casque.
